# EUROPÄISCHE PATENTSCHRIFT

(11) **EP 1 796 570 B1**
(45) Veröffentlichungstag und Bekanntmachung des Hinweises auf die Patenterteilung: **21.05.2008**
(21) Anmeldenummer: 04761923.4
(22) Anmeldetag: 15.09.2004
(51) Int. Cl.: A61B 19/00

(54) **KALIBRIERVORRICHTUNG**
CALIBRATING DEVICE
DISPOSITIF D'ETALONNAGE

(43) Veröffentlichungstag der Anmeldung: 20.06.2007
(73) Patentinhaber: AO Technology AG, 7002 Chur (CH)
(72) Erfinder: LANGLOTZ, Frank, CH-4800 Zofingen (CH); ROHRER, Urs, CH-3210 Kerzers (CH)
(74) Vertreter: Lusuardi, Werther
(86) Internationale Anmeldenummer: PCT/CH2004/000583
(87) Internationale Veröffentlichungsnummer: WO 2006/029541

(56) Entgegenhaltungen:
- WO-A-02/061371
- US-A1- 2003 040 879
- US-A1- 2004 039 402
- US-A1- 2004 167 654
- US-B1- 6 306 126

## Beschreibung

Die Erfindung bezieht sich auf eine Vorrichtung zur Kalibrierung von geometrischen Abmessungen, der Orientierung und der Position von chirurgischen Instrumenten im Raum, gemäss dem Oberbegriff des Patentanspruchs 1.

Solche Vorrichtungen eignen sich für die Kalibrierung von geometrischen Abmessungen, insbesondere des Durchmessers, sowie der Lage und/oder Orientierung von chirurgischen Instrumenten im Raum. Die Kalibrierung der Lage und Orientierung ist vor allem für chirurgische Instrumente notwendig, welche in der Computerunterstützten Chirurgie eingesetzt werden, mittels welcher die Lage und Orientierung verschiedener chirurgischer Instrumente während der Operation mittels eines eine Positionserfassungsvorrichtung umfassenden chirurgischen Navigationssystemes vermessen wird. Durch ein solches Navigationssystem ist nur die Position von am Instrument oder an einer das Instrument umfassenden Maschine, beispielsweise einer Bohrmaschine, angebrachten Marker bestimmbar. Beispielsweise bei Bohrvorgängen, wo der Bohrer, wenn er in-situ in die Bohrmaschine eingespannt wird, in einer vorgängig nicht bekannten Position relativ zur Bohrmaschine und den Markern eingespannt wird, ist daher eine intraoperative Kalibrierung unumgänglich.

Aus der EP-A 0 904 735 MESSNER ist eine gattungsgemässe Vorrichtung bekannt. Diese bekannte Kalibriervorrichtung gestattet neben dem Bestimmen der Lage einer Instrumentenspitze auch die Messung des Instrumentendurchmessers mittels zwei linear relativ zueinander verschiebbaren Backen. Die Backen müssen vor der Messung derart relativ zueinander verschoben werden, dass sie präzis auf ihrer ganzen zur Längsachse des chirurgischen Instrumentes parallelen Länge an der Mantelfläche des chirurgischen Instrumentes anliegen. Nachteilig an dieser bekannten Vorrichtung ist, dass bei nicht korrekt auf der gesamten Länge anliegenden Backen Messfehler auftreten können.

Eine weitere Kalibriervorrichtung ist bekannt aus US2004/0167654

Hier will die Erfindung Abhilfe schaffen. Der Erfindung liegt die Aufgabe zugrunde, eine Vorrichtung zu schaffen, welche die Messung von relevanten geometrischen Abmessungen, insbesondere des Durchmessers, der Breite oder der Höhe von longitudinalen, prismatischen oder zylindrischen Objekten mit einer zentralsymmetrischen Querschnittsfläche ermöglicht, ohne dass ein Verschieben der Backen notwendig ist.

Die Erfindung löst die gestellte Aufgabe mit einer Vorrichtung zur Kalibrierung von geometrischen Abmessungen und der Orientierung von chirurgischen Instrumenten im Raum, welche die Merkmale des Anspruchs 1 aufweist.

Die durch die Erfindung erreichten Vorteile sind im wesentlichen darin zu sehen, dass dank der erfindungsgemässen Vorrichtung:
- unterschiedliche zylindrische oder prismatische, chirurgische Instrumente mit kreis- oder rechteckförmiger Querschnittsfläche in einer bezüglich der Position der Marker definierten Lage an der Andockstation andockbar sind;
- zylindrische oder prismatische, chirurgische Instrumente mit anderen als kreis- oder rechteckförmigen Querschnittsflächen, d.h. beispielsweise ovaler oder elliptischer Querschnittsfläche in einer durch den Operateur definierten Lage an der Andockstation andockbar sind;
- der Durchmesser von kreiszylindrischen Objekten oder die Breite oder Höhe von Objekten mit einer anderen Querschnittsfläche vermessbar sind, ohne dass ein Verschieben der Backen notwendig ist;
- die Orientierung unterschiedlicher prismatischer oder zylindrischer, chirurgischer Instrumente im Raum vermessbar ist;
- wegen der vorzugsweise am Instrumentengriff, d.h. vom Instrumentenschaft axial entfernt angebrachten Marker eine Verlängerung der den Winkel α einschliessenden Schenkel entsteht, so dass eine geringe Veränderung des Winkels α eine zusätzliche Vergrösserung der Abstände zwischen den Markern am Instrument und denjenigen an der Vorrichtung entsteht, womit eine höhere Kalibriergenauigkeit als bei linear verschiebbaren Backen erreichbar ist;
- die Backen unbewegbar mit der Andockstation verbunden sind, so dass kein Verkanten oder Verklemmen bei der Messung stattfinden kann.

Weitere vorteilhafte Ausgestaltungen der Erfindung sind in den abhängigen Ansprüchen gekennzeichnet.

In einer bevorzugten Ausführungsform sind die Backen mindestens an ihren sich gegenüberliegenden Oberflächenpartien doppelkegelförmige ausgebildet und umfassen axial mittig eine quer zur Zentralachse eindringende Einkerbung. Der Vorteil dieser Ausgestaltung liegt im wesentlichen darin, dass die gekerbte Form der Backen hilft, ein eingelegtes Instrument in einer definitiven Lage zu positionieren. Vorzugsweise sind die Einkerbungen der Backen zueinander fluchtend ausgebildet, wodurch der weitere zur Messung gewünschte Effekt, dass mit steigendem Durchmesser eine Zunahme des Winkels α erreichbar ist, vergrössert wird, da sich die beiden Auflagepunkte des Instrumentes mit steigendem Durchmesser weiter von den Zentralachsen der Backen entfernen.

Ferner sind die Einkerbungen vorzugsweise V-förmig ausgebildet und weisen quer zur Zentralachse eine Tiefe T auf.

In einer weiteren Ausführungsform umfasst die Vorrichtung zusätzlich Positioniermittel, welche zur exakten Positionierung der vorderen Enden von unterschiedlichen chirurgischen Instrumenten relativ zur Andockstation geeignet sind. Damit sind die Vorteile erreichbar, dass die Position des vorderen Endes, beispielsweise einer Bohrerspitze mit derselben Vorrichtung erfassbar ist. Ferner können die Position des vorderen Endes eines Instrumentes und seine Achse in unabhängigen Schritten nacheinander kalibriert werden. In der in der EP-A 0 904 735 MESSNER offenbarten Vorrichtung muss in einem einzigen Arbeitsschritt sichergestellt werden, dass das Instrument bis zum Anschlag in die Kalibriervorrichtung eingeschoben wurde und die beiden Backen korrekt am Instrumenten anliegen.

In wiederum einer anderen Ausführungsform sind die Backen relativ zueinander um mindestens zwischen 100 mm und 300 mm beabstandet, so dass durch den grossen Abstand der Backen bei der Winkelmessung eine hohe Genauigkeit bei der Kalibrierung der Lage der Instrumentenlängsachse ermöglicht wird. Ferner sind die Backen relativ zur Andockstation nicht bewegbar.

In wiederum einer weiteren Ausführungsform umfasst die Vorrichtung einen Drehteller mit einer quer zur Oberfläche verlaufenden Drehachse und zwei über die Oberfläche vorstehende Klemmbacken, so dass das chirurgische Instrument mittels der Klemmbacken auf dem Drehteller lösbar an der Vorrichtung festklemmbar ist.

In einer anderen Ausführungsform ist an der Andockstation eine Torsionsfeder angeordnet, mittels welcher der Drehteller in eine erste Drehrichtung um die Drehachse gedrückt wird. Damit ist erreichbar, dass durch die Torisonsfeder ein schräg zwischen den Backen eingeführtes chirurgisches Instrument selbsttätig und ohne manuelle Kraftaufwendung an die Backen pressbar ist oder bei umgekehrtem Drehsinn ein chirurgisches Instrument zwischen die Backen einführbar ist. Des Weiteren wird gewährleistet, dass das Instrument an beide Backen gepresst wird und in der gewünschten, definierten Position anliegt.

Vorzugsweise ist der Drehteller mittels eines Bedienungselementes gegen die Federkraft in einer zweiten Drehrichtung um die Drehachse bewegbar. Damit ist der Vorteil erreichbar, dass der Drehteller mittels des Bedienungselementes so drehbar ist, dass ein chirurgisches Instrument zwischen die Backen eingeführt werden kann oder bei umgekehrtem Drehsinn gegen die Backen pressbar ist. Indem die eine Drehrichtung durch Federkraft und die andere durch Handkraft unterstützt wird, ist zudem eine einfache Handbedienung der Vorrichtung gewährleistet.

Vorzugsweise sind die Klemmbacken so auf dem Drehteller angeordnet, dass sie zentral von einer die Drehachse schneidenden Geraden durchdrungen werden, wobei bei einer Rotation des Drehtellers in der ersten Drehrichtung der zwischen der Geraden und der Referenzgeraden eingeschlossene Winkel β verringert wird, so dass ein zwischen den Backen eingeführtes chirurgisches Instrument gegen die Backen pressbar ist.

In wiederum einer anderen Ausführungsform ist zwischen dem Bedienungselement und dem Drehteller ein Zahnradgetriebe eingesetzt.

Vorzugsweise umfassen die Positioniermittel Vertiefungen, welche eine voneinander unterschiedliche Geometrie, insbesondere unterschiedliche Querschnittsflächen aufweisen.

In einer weiteren Ausführungsform stehen die Zentralachsen der Backen senkrecht auf der Oberfläche. Ferner sind die Backen mindestens auf den sich gegenüberliegenden Oberflächenpartien kreiszylindrisch ausgestaltet, während die Oberfläche eben ausgebildet ist. Die hiermit erreichbaren Vorteile liegen darin, dass flache Instrumente, beispielsweise Meissel oder Sägeblätter auf der Oberfläche aufgelegt werden können, so dass kein Verkanten zwischen den Backen stattfinden kann.

Das Verfahren zur Kalibrierung von geometrischen Abmessungen von chirurgischen Instrumenten sowohl als auch der Orientierung derselben im Raum gemäß der Ansprüche 16 und 17 beinhaltet im wesentlichen die Schritte:
A) Einführen eines chirurgischen Instrumentes in einen U-artigen Durchgang, welcher durch die Oberfläche der Andockstation und zwei zur Andockstation zählenden, kreiszylindrischen oder doppelkegelförmigen Backen gebildet wird;
B) Drehen des chirurgischen Instrumentes derart, dass der Winkel α, welcher von der Längsachse des chirurgischen Instrumentes und einer durch die Form der Backen oder die Form der Backen und die Oberfläche definierten Referenzgeraden eingeschlossen wird, so weit verringert wird, bis die Mantelfläche des chirurgischen Instrumenten an den Seitenwänden der Backen in einer durch die Form der Backen oder die Form der Backen und die Oberfläche definierten Orientierung zur Anlage kommt;
C) Messen der räumlichen Position der Marker, welche an einem an der Andockstation befestigten Referenzmittel und an einem am chirurgischen Instrument befestigten Referenzmittel befestigt sind, mittels einer Positionserfassungsvorrichtung;
D) Ermitteln des Winkels α aus den gemessenen Positionen aller Marker mittels eines Computers; und
E) Ermitteln der sich senkrecht zu den Zentralachsen der Backen erstreckenden Abmessung des chirurgischen Instrumentes aus dem gemessenen Winkel α, der bekannten Geometrie der Backen relativ zu den Markern am Referenzmittel an der Andockstation mittels des Computers.
   In einer bevorzugten Ausführung umfasst das Verfahren zusätzlich die folgenden Schritte:
F) Einführen des vorderen Endes eines chirurgischen Instrumentes in eine der an der Andockstation angebrachten Vertiefungen, wobei die Position des Grundes der Vertiefung relativ zu den Markern am Referenzmittel an der Andockstation bekannt ist; und
G) Messen der räumlichen Position der Marker, welche an dem an der Andockstation befestigten Referenzmittel und an dem am chirurgischen Instrument befestigten Referenzmittel befestigt sind, mittels der Positionserfassungsvorrichtung;
H) Ermitteln der räumlichen Position des vorderen Endes des chirurgischen Instrumentes aus den gemessenen Positionen aller Marker mittels des Computers.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht einer Ausführungsform der erfindungsgemässen Vorrichtung zusammen mit einem chirurgischen Navigationssystem;
Fig. 2 eine Aufsicht auf eine Ausführungsform der erfindungsgemässen Vorrichtung;
Fig. 3 einen Längsschnitt durch die in Fig. 2 dargestellte Ausführungsform der erfindungsgemässen Vorrichtung; und
Fig. 4 eine perspektivische Ansicht der in den Fig. 2 und 3 dargestellten Ausführungsform der erfindungsgemässen Vorrichtung.

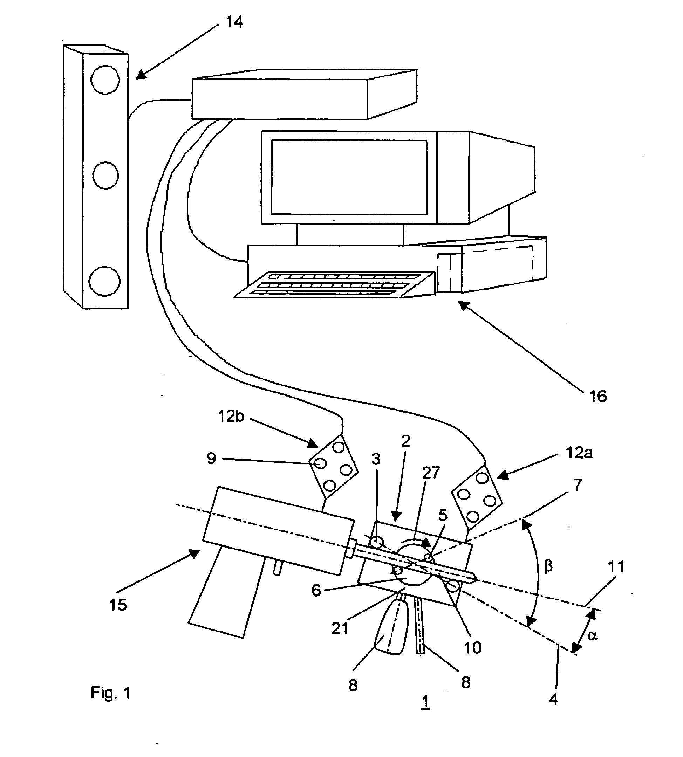

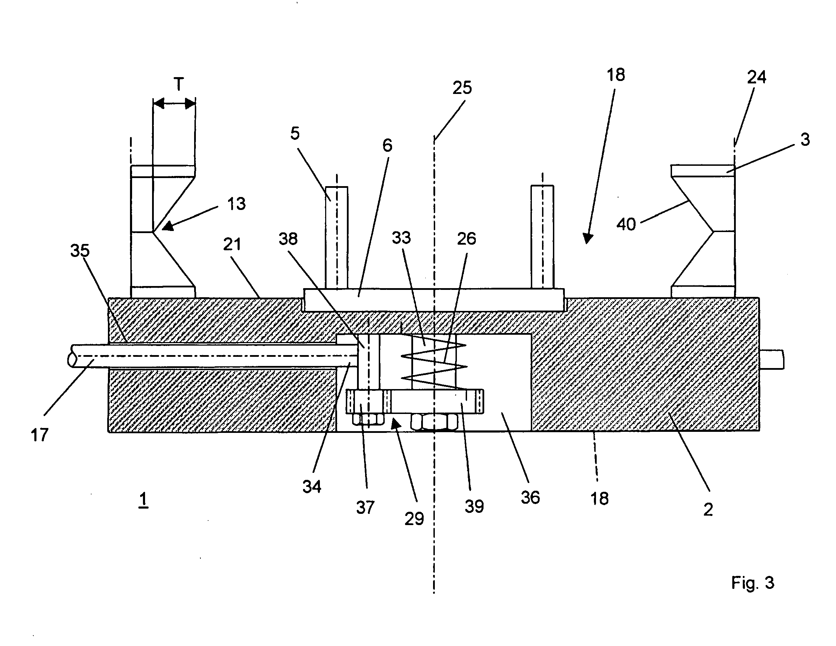

Fig. 1 zeigt eine Ausführungsform der Vorrichtung 1, welche im wesentlichen durch eine Andockstation 2 mit zwei Backen 3 und einem Referenzmittel 12a realisiert ist, zusammen mit einem koaxial zu seiner Längsachse 11 in einer Antriebsmaschine 15 eingespannten Bohrer als chirurgisches Instrument 10 sowie mit einer Positionserfassungsvorrichtung 14 und mit einem Computer 16. Die Backen 3 und die Oberfläche 21 schliessen einen U-artigen, zur Durchführung eines longitudinalen chirurgischen Instrumentes 10 geeigneten Durchgang 18 (Fig. 3) ein. Die kreiszylindrischen Backen 3 weisen parallele, senkrecht auf der ebenen Oberfläche 21 der Andockstation 2 stehende Zentralachsen 24 auf. Das chirurgische Instrument 10, d.h. der Bohrer ist zwischen den Backen 3 durchgeführt und liegt an zwei Stellen mit seiner zur Längsachse 11 konzentrischen Mantelfläche an den Backen 3 an und auf der Oberfläche 21 auf, wobei die Längsachse 11 mit einer die beiden Zentralachsen 24 (Fig. 3) der Backen 3 rechtwinklig schneidenden Referenzgeraden 4 ein Winkel α einschliesst. An der Antriebsmaschine 15 ist ein weiteres Referenzmittel 12b angebracht. Beide Referenzmittel 12 sind mit je vier elektromagnetisch oder akustisch erfassbaren Markern 9 versehen, so dass mittels der Positionserfassungsvorrichtung 14 die räumliche Position der Marker 9 vermessbar ist. Durch die Vermessung der räumlichen Position der Marker 9 kann anschliessend mittels des Computers 16 die Lage der Längsachse 11 und der Referenzgeraden 4 sowie der dazwischen eingeschlossene Winkel α ermittelt werden. Mit den bekannten Geometrien der zwei Backen 3 und des chirurgischen Instrumentes 10 lässt sich bei bekanntem Winkel α der Durchmesser des hier als Bohrer ausgestalteten chirurgischen Instrumentes 10 ermitteln. Bei anders ausgestalteten chirurgischen Instrumenten 10 wird die zwischen den Backen 3 eingeschlossene, senkrecht zu den Zentralachsen 24 stehende Abmessung ermittelt. Die Backen 3 sind fest an der Andockstation 2 angeordnet und hier kreiszylindrisch ausgebildet. Ferner umfasst die Andockstation 2 einen Drehteller 6 mit einer senkrecht zur Oberfläche 21 verlaufenden Drehachse 25, welcher zwei über die Oberfläche 21 vorstehende Klemmbacken 5 umfasst. Der Drehteller 6 ist mittels eines Bedienungshebels 17, welcher neben dem fest mit der Andockstation 2 verbundenen Handgriff 8 angeordnet ist, gegen die Federkraft einer Torsionsfeder 26 (Fig. 3) um die Drehachse 25 rotierbar. Bei nicht kraftbeaufschlagtem Bedienungshebel 17 wird der Drehteller 25 durch die Federkraft der Torsionsfeder 26 (Fig. 3) in einer ersten Drehrichtung 27 bewegt, so dass das zwischen den Backen 3 durchgeführte chirurgische Instrument 10 durch die auf dem Drehteller 6 angeordneten Klemmbacken 5 gegen die Backen 3 gepresst wird. Wenn der Bedienungshebel 17 gegen den Handgriff 8 gepresst wird, wird der Drehteller 6 in der entgegengesetzten, zweiten Drehrichtung 28 bewegt, so dass ein chirurgisches Instrument 10, ohne durch die Klemmbacken 5 behindert zu werden, in die Andockstation 2 einführbar ist. Der Drehteller 6 ist hier zur Drehachse 25 konzentrisch kreisrund ausgebildet, wobei die beiden Klemmbacken 5 diametral angeordnet sind.

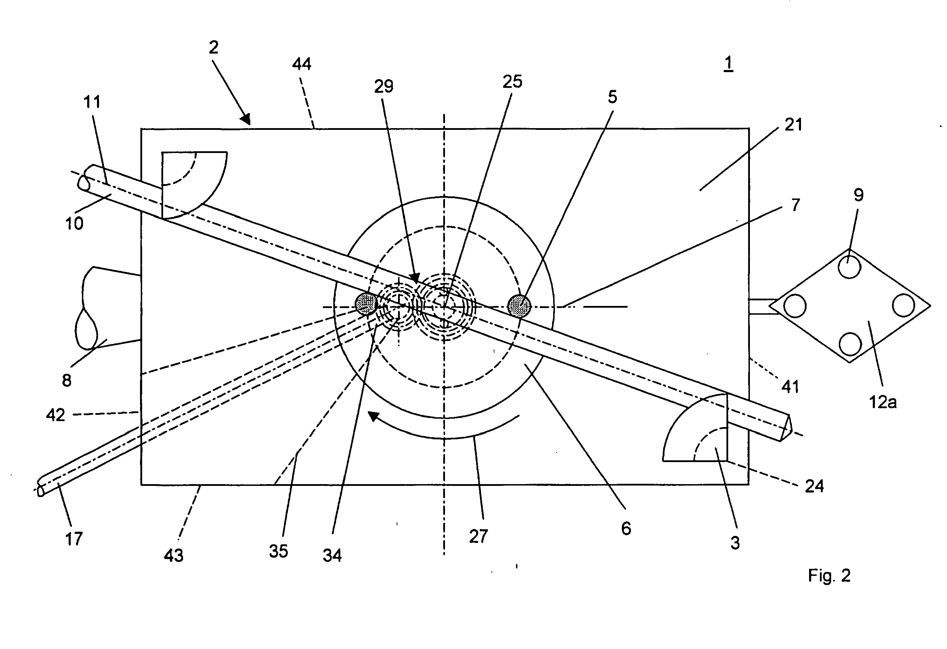

In den Fig. 2 und 3 ist eine Ausführungsform der Vorrichtung 1 dargestellt, deren quaderartige Andockstation 2 zwei in einer Diagonalen auf der Oberfläche 21 stehende Backen 3 umfasst. Die Backen 3 sind auf den sich gegenüberliegenden Oberflächenpartien doppelkegelförmig ausgebildet und somit axial mittig mit einer V-förmigen, quer zu den Zentralachsen 24 in die Backen 3 eindringenden Einkerbungen 13 zur definierten Positionierung eines chirurgischen Instrumentes 10 an den Backen 3 versehen. Die Zentralachsen 24 werden durch die Zentren von zwei kreissektorförmigen Kegelquerschnittsflächen pro Backe 3 definiert. Die Einkerbungen 13 an den Seitenwänden 40 weisen quer zu den Zentralachsen 24 gemessen eine Tiefe T auf und fluchten zueinander. Das chirurgische Instrument 10 ist derart zwischen die Backen 3 eingeführt, dass seine Längsachse 11 zwischen den Backen 3 durchgeht und die zur Längsachse 11 konzentrische Mantelfläche des chirurgischen Instrumentes 10 an je zwei Punkten in jeder der zwei Einkerbungen 13 an den zwei Seitenwänden 40 der Backen 3 anliegt. Ferner ist zwischen der Achse 33 des Drehtellers 6 und dem vorderen Ende 34 des Bedienungshebels 17 ein Zahnradgetriebe 29 angeordnet. Die hier dargestellte Andockstation 2 weist vier Seitenflächen 41 ;42;43;44 auf, wobei an der ersten Seitenfläche 41 die Referenzmittel 12 mit vier elektromagnetisch oder akustisch erfassbaren Markern 9 angeordnet sind. Auf der gegenüberliegenden, zweiten Seitenfläche 42 ist der Handgriff 8 zusammen mit dem Bedienungshebel 17 angeordnet. Der Bedienungshebel 17 wird durch eine Öffnung 35 bis in den zentralen, gegen die Grundfläche 18 der Andockstation 2 offenen Hohlraum 36 geführt und ist mit seinem vorderen Ende 34 an der rotierbar mit der Andockstation 2 verbundenen Achse 38 des ersten Zahnrades 37 befestigt. Das erste Zahnrad 37 steht mit einem zweiten Zahnrad 39 im Eingriff, welches an der ebenfalls mit der Andockstation 2 rotierbar verbundenen Achse 33 des Drehtellers 6 befestigt ist. Die Achse 33 des Drehteller 6 verläuft koaxial zur Drehachse 25 des Drehtellers 6 und steht senkrecht zur Oberfläche 21. Ferner sind auf dem Drehteller 6 zwei über die Oberfläche 21 vorstehende Klemmbacken 5 angeordnet. Der Drehteller 6 ist in seiner zur Drehachse 25 orthogonalen Querschnittsfläche kreisrund ausgebildet, wobei die zwei Klemmbacken 5 auf einer Diagonalen angeordnet sind. Ferner ist zwischen der Andockstation 2 und dem zweiten Zahnrad 39 eine zur Drehachse 25 konzentrische Torsionsfeder 26 angeordnet, mittels welcher der Drehteller 6 in die erste Drehrichtung 27 gedrückt wird. Mittels des Bedienungshebels 8 ist der Drehteller 6 gegen die Federkraft in der zweiten Drehrichtung bewegbar. Durch die Federkraft der Torsionsfeder 26 wird erreicht, dass der Drehtellers 6 in der ersten Drehrichtung 27 so bewegt wird, dass der zwischen der Geraden 7, welche die Drehachse 25 schneidet und die Klemmbacken 5 zentral durchdringt, und der Referenzgeraden 4 eingeschlossene Winkel β (Fig. 1) verringert wird, so dass ein zwischen den Backen 3 eingeführtes chirurgisches Instrument 10 gegen die Backen 3 gepresst wird.

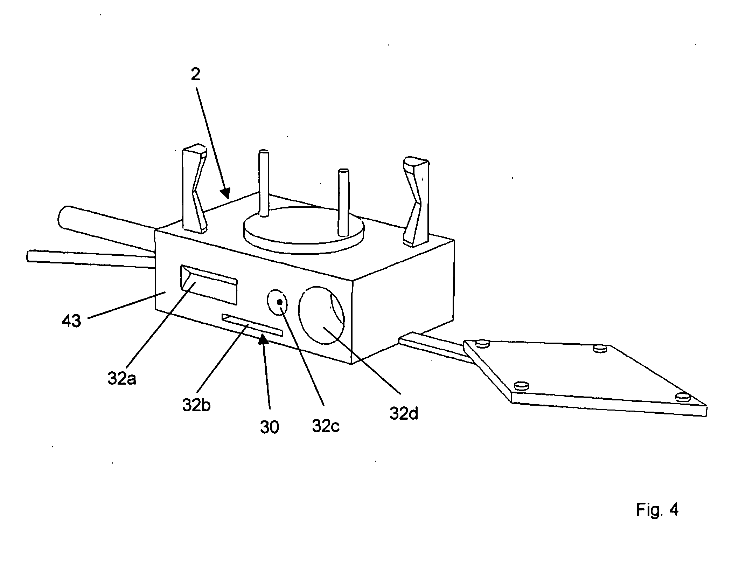

In Fig. 4 ist eine Ausführungsform dargestellt, welche an einer dritten Seitenfläche 43 der Andockstation 2 angeordnete Positioniermittel 30 zur Positionierung der vorderen Enden 31 (Fig. 2) von unterschiedlichen chirurgischen Instrumenten 10 relativ zur Andockstation 2 umfasst. Diese Positioniermittel 30 umfassen Vertiefungen 32, welche eine voneinander unterschiedliche Geometrie zur definierten Aufnahme von unterschiedlichen chirurgischen Instrumenten 10 (Fig. 1;2) aufweisen. Insbesondere sind die Vertiefung 32a;32b;32c;32d so ausgebildet, dass:
- die Vertiefung 32a eine zur dritten Seitenfläche 43 parallele rechteckförmige Eintrittsöffnung aufweist und sich von den langen Seiten der Eintrittsöffnung gegen den Grund der Vertiefung 32a zuspitzt, so dass sie zur Aufnahme des vorderen Endes beispielsweise eines flachen Meissels geeignet ist;
- die Vertiefung 32b als Schlitz mit ebenem Grund der Vertiefung 32b ausgebildet ist, so dass sie zur Aufnahme des vorderen Endes beispielsweise eines Sägeblattes geeignet ist;
- die Vertiefung 32c ist konisch ausgebildet und zur Zentrierung der Spitze beispielsweise eines Pointers geeignet; und
- die Vertiefung 32d ist als kreiszylindrische Bohrung mit einem ebenen Grund ausgebildet, so dass sie zur Aufnahme von zylindrischen Instrumenten mit verschiedenen Spitzen geeignet ist.

## Patentansprüche

1. Vorrichtung (1) zur Kalibrierung von geometrischen Abmessungen von chirurgischen Instrumenten (10) und der Orientierung derselben im Raum, umfassend
A) eine zwei Backen (3) und eine Oberfläche (21) aufweisende Andockstation (2); und
B) mindestens drei fix an der Andockstation (2) angeordnete, bezüglich ihrer Position im Raum elektromagnetisch oder akustisch vermessbaren Markern (9) zur Bestimmung der Position und Orientierung der Vorrichtung (1) im Raum mittels einer Positionserfassungsvorrichtung (14),
C) die Backen (3) relativ zur Oberfläche (21) der Andockstation (2) feststehen und je eine Seitenwand (40) aufweisen, so dass die Oberfläche (21) und die Seitenwände (40) der zwei Backen (3) einen U-artigen Durchgang (18) einschliessen; wobei
D) die zwei Backen (3) mindestens auf den sich gegenüberliegenden Oberflächenpartien kreiszylindrisch, hyperboloid- oder doppelkegelförmig ausgebildet sind, so dass ein zwischen die Backen (3) eingeführtes chirurgisches Instrument (10) quer zu seiner Längsachse (11) an mindestens je einem Auflagepunkt an jeder Backe (3) zur Anlage bringbar ist; und
E) die zwei Seitenwände (40) oder die Oberfläche (21) eine Form aufweisen, weiche mindestens zwei weitere Auflagepunkte für ein zwischen die Backen (3) eingeführtes chirurgisches Instrument (10) quer zu dessen Längsachse (11) umfassen, so dass ein relativ zu den Markern (9) definiertes, seitliches Andocken eines zwischen den Backen (3) durchgeführten chirurgischen Instrumentes (10) ermöglicht wird;
**dadurch gekennzeichnet, dass**
F) die Vorrichtung (1) einen Drehteller (6) mit, einer quer zur Oberfläche (21) verlaufenden Drehachse (25) und zwei über die Oberfläche (21) vorstehende Klemmbacken (5) umfasst.

2. Vorrichtung (1) nach Anspruch 1, **dadurch gekennzeichnet, dass** die zwei Backen (3) mindestens an ihren sich gegenüberliegenden Oberflächenpartien doppelkegelförmig, axial mittig eine V-förmige Einkerbung (13) umfassend ausgebildet sind, und eine durch die Kreiszentren der kreis- oder kreissektorförmigen Kegelquerschnittsflächen definierte Zentralachse (24) aufweisen.

3. Vorrichtung (1) nach Anspruch 2, **dadurch gekennzeichnet, dass** die Einkerbungen (13) der zwei Backen (3) eine Tiefe T aufweisen und zueinander fluchtend ausgebildet sind.

4. Vorrichtung (1) nach Anspruch 3, **dadurch gekennzeichnet, dass** der Winkel α zwischen einer die Seitenwände (40) in den Einkerbungen (13) in der Tiefe T durchdringenden und die Zentralachsen (24) schneidenden Referenzgeraden (4) und der Längsachse (11) eines angedockten chirurgischen Instrumentes (10) α < 90° ist.

5. Vorrichtung (1) nach einem der Ansprüche 1 bis 4, **dadurch gekennzeichnet, dass** sie zusätzlich Positioniermittel (30) zur Positionierung der vorderen Enden (31) von unterschiedlichen chirurgischen Instrumenten (10) relativ zur Andockstation (2) umfasst.

6. Vorrichtung (1) nach einem der Ansprüche 1 bis 5, **dadurch gekennzeichnet, dass** die Backen (3) relativ zueinander einen Abstand mindestens zwischen 100 mm und 300 mm aufweisen.

7. Vorrichtung (1) nach einem der Ansprüche 1 bis 6, **dadurch gekennzeichnet, dass** die Backen (3) Zentralachsen (24) aufweisen, welche zueinander parallel sind.

8. Vorrichtung (1) nach einem der Ansprüche 1 bis 7, **dadurch gekennzeichnet, dass** an der Andockstation (2) eine Torsionsfeder (26) angeordnet ist, mittels weicher der Drehteller (6) in eine erste Drehrichtung (27) um die Drehachse (25) gedrückt wird.

9. Vorrichtung (1) nach Anspruch 8, **dadurch gekennzeichnet, dass** der Drehteller (6) mittels eines Bedienungselementes (8) gegen die Federkraft in einer zweiten Drehrichtung um die Drehachse (25) bewegbar ist.

10. Vorrichtung (1) nach einem der Ansprüche 1 bis 9, **dadurch gekennzeichnet, dass** die Klemmbacken (5) so auf dem Drehteller (6) angeordnet sind, dass sie zentral von einer die Drehachse (25) schneidenden Geraden (7) durchdrungen werden.

11. Vorrichtung (1) nach Anspruch 10, **dadurch gekennzeichnet, dass** bei einer Rotation des Drehtellers. (6) in der ersten Drehrichtung (27) der zwischen der Geraden (7) und der Referenzgeraden (4) eingeschlossene Winkel β verringert wird, so dass ein zwischen den Backen (3) eingeführtes chirurgisches Instrument (10) gegen die Backen (3) pressbar ist.

12. Vorrichtung (1) nach einem der Ansprüche 9 bis 11, **dadurch gekennzeichnet, dass** zwischen dem Bedienungselement (8) und dem Drehteller (6) ein Zahnradgetriebe (29) angeordnet ist.

13. Vorrichtung (1) nach einem der Ansprüche 5 bis 12, **dadurch gekennzeichnet, dass** die Positioniermittel (30) Vertiefungen (32) umfassen, welche eine voneinander unterschiedliche Geometrie aufweisen.

14. Vorrichtung (1) nach Anspruch 13, **dadurch gekennzeichnet, dass** die Vertiefungen (32) unterschiedliche Querschnittsflächen aufweisen.

15. Vorrichtung (1) nach einem der Ansprüche 1 und 5 bis 14, **dadurch gekennzeichnet, dass** die Oberfläche (21) eben ausgestattet ist und die Backen (3) mindestens in den sich gegenüberliegenden Oberflächenpartien kreiszylindrisch sind.

16. Verfahren zur Kalibrierung von geometrischen Abmessungen von chirurgischen Instrumenten (10) sowohl als auch der Orientierung derselben im Raum mittels des Vorrichtung nach einem der Ansprüche 1-15 mit den Schritten
A) Einführen eines chirurgischen Instrumentes (10) in einen U-artigen Durchgang (18), welcher durch die Oberfläche (21) einer Andockstation (2) und zwei zur Andockstation (2) zählenden, kreiszylindrischen oder doppelkegelförmigen Backen (3) gebildet wird;
B) Drehen des chirurgischen Instrumentes (10) derart, dass der Winkel α, welcher von der Längsachse (11) des chirurgischen Instrumentes (10) und einer durch die Form der Backen (3) oder die Form der Backen (3) und die Oberfläche (21) definierten Referenzgeraden (4) eingeschlossen wird, so weit verringert wird, bis die Mantelfläche des chirurgischen Instrumenten (10) an den Seitenwänden (40) der Backen (3) in einer. durch die Form der Backen (3) oder die Form der Backen (3) und die Oberfläche (21) definierten Orientierung zur Anlage kommt;
C) Pressen des chirurgischen Instruments (10) gegen die Backen (3) mittels den auf dem Drehteller (6) angeordneten Klemmbacken (5);
D) Messen der räumlichen Position der Marker (9), welche an der Andockstation (2) und am chirurgischen Instrument (10) befestigt sind, mittels einer Positionserfassungsvorrichtung (14);
E) Ermitteln des Winkels α aus den gemessenen Positionen aller Marker (9) mittels eines Computers (16); und
F) Ermitteln des sich senkrecht zu den Zentralachsen (24) der Backen (3) erstreckenden Durchmessers oder der Breite des chirurgischen Instrumentes (10) aus dem gemessenen Winkel α, der bekannten Geometrie der Backen (3) relativ zu den Markern (9) an der Andockstation (2) mittels des Computers (16).

17. Verfahren nach Anspruch 16, **dadurch gekennzeichnet, dass** es zusätzlich die folgenden Schritte umfasst:
G) Einführen des vorderen Endes (31) eines chirurgischen Instrumentes (10) in eine der an der Andockstation (2) angebrachten Vertiefungen (32), wobei die Position des Grundes der Vertiefung (32) relativ zu den Markern (9) am Referenzmittel (12a) an der Andockstation (2) bekannt ist; und
H) Messen, der räumlichen Position der Marker (9), welche an der Andockstation (2) und am chirurgischen Instrument (10) befestigt sind, mittels der Positionserfassungsvorrichtung (14);
I) Ermitteln der räumlichen Position des vorderen Endes (31) des chirurgischen Instrumentes (10) aus den gemessenen Positionen aller Marker (9) mittels des Computers (16).

## Claims

1. Device (1) for calibrating geometrical dimensions of surgical tools (10) and the orientation of the same in space, comprising:
A) a docking station (2) having two jaws (3) and a surface (21); and
B) at least three marking indicators (9) arranged on the docking station (2) in a fixed manner and measurable in reference to their spatial position electromagnetically or acoustically, in order to determine the position and orientation of the device (1) in space through a position detecting device (14),
C) the jaws (3) are stationary with respect to the surface (21) of the docking station (2) and have one lateral wall (40) each, so that the surface (21) and the lateral walls (40) of the two jaws (3) encompass a U-shaped passage (18); where
D) the two jaws (3) are, at least on the surface portions opposite to each other, conformed in a circular cylindrical, hyperboloid or double-cone shaped fashion, so that a surgical tool (10) which has been inserted between the jaws (3) can be laid down on at least one contact point of each jaw (3) across its longitudinal axis (11); and
E) the two lateral walls (40) or the surface (21) have a form comprising at least two additional points of contact for a surgical tool (10) inserted between the jaws (3) across its longitudinal axis (11), so as allow a lateral docking, defined with respect to the marking indicators (9), of a surgical tool (10) passed between the jaws (3),
**characterized in that**
F) the device (1) comprises a rotary table (6) with an axis of rotation (25) running across the surface (21) and two clamping jaws (5) projecting over the surface (21).

2. Device (1) according to claim 1, **characterized in that** the two jaws (3) are, at least on their surface portions opposite to each other, conformed in a double-conical form comprising axially centric a V-shaped indentation (13) and having a central axis (24) defined by the centres of the circular or circle sector-shaped cone cross sectional surfaces.

3. Device (1) according to claim 1, **characterized in that** the indentations (13) of the two jaws (3) have a depth T and are conformed in a manner aligned to each other.

4. Device (1) according to the claims 2 or 3, **characterized in that** the angle α between a reference straight line (4) that penetrate the lateral walls (40) in the indentations (13) at the depth T and cross the central axes (24) and the longitudinal axis (11) of a docked surgical tool (10) is α < 90°.

5. Device (1) according to one of the claims from 1 to 4, **characterized in that** it additionally comprises positioning means (30) to position the front ends (31) of different surgical tools (10) with respect to the docking station (2).

6. Device (1) according to one of the claims from 1 to 5, **characterized in that** the jaws (3) have a distance from each other of at least between 100 mm and 300 mm.

7. Device (1) according to one of the claims from 1 to 6, **characterized in that** the jaws (3) have central axes (24) that are parallel to each other.

8. Device (1) according to one of the claims from 1 to 7, **characterized in that** a torsion spring (26) is arranged on the docking station (2), by means of which the rotary table (6) is pressed in a first rotating direction (27) around the axis of rotation (25).

9. Device (1) according to claim 8, **characterized in that** the rotary table (6) can be moved through an operating element (8) against the force of the spring in a second direction of rotation around the axis of rotation (25).

10. Device (1) according to one of the claims from 1 to 9, **characterized in that** the clamping jaws (5) are arranged on the rotary table (6) so as to be centrally penetrated by a straight line (7) crossing the axis of rotation (25).

11. Device (1) according to claim 10, **characterized in that** at a rotation of the rotary table (6) in the first direction of rotation (27), the angle β enclosed between the straight line (7) and the reference straight line (4) is reduced, so that a surgical tool (10) inserted between the jaws (3) can be pressed against the jaws (3).

12. Device (1) according to one of the claims from 9 to 11, **characterized in that** a toothed-wheel gearing (29) is arranged between the operating element (8) and the rotary table (6).

13. Device (1) according to one of the claims from 5 to 12, **characterized in that** the positioning means (30) comprise depressions (30) having geometries differing from each other.

14. Device (1) according to claim 13, **characterized in that** the depressions (32) have different cross sectional areas.

15. Device (1) according to one of the claims 1 and from 5 to 14, **characterized in that** the surface (21) is conformed in a flat manner and that the jaws (3) are of a round cylindrical form at least in the surface portions opposite to each other.

16. Process for calibrating geometrical measurements of surgical tools (10) as well as for orienting the same in space by means of the device according to one of the claims 1 to 15, with the following steps:
A) inserting a surgical tool (10) into a U-shaped passage (18), formed by the surface (21) of a docking station (2) and two circular cylindrical or double cone-shaped jaws (3) belonging to the docking station (2);
B) rotating the surgical tool (10) so that the angle α, which is enclosed by the longitudinal axis (11) of the surgical tool (10) and by a reference straight line (4) defined by the shape of the jaws (3) or the shape of the jaws (3) and the surface (21), is reduced to the point that the peripheral surface of the surgical tool (10) comes to rest against the lateral walls (40) of the jaws (3) at an orientation defined by the form of the jaws (3), or the form of the jaws (3) and the surface (21).
C) pressing the surgical tool (10) against the jaws (3) by means of the clamping jaws (5) arranged on the rotary table (6);
D) measuring the spatial position of the marking indicators (9) fastened to the docking station (2) and to the surgical tool (10), through a position detecting device (14);
E) determining the angle α from the measured positions of all marking indicators (9) through a computer (9); and
F) determining the diameter extending perpendicularly to the central axes (24) of the jaws (3) or the width of the surgical tool (10) from the measured angle α and the known geometry of the jaws (3) with respect to the marking indicators (9) on the docking station (2) through a computer (16).

17. Process according to claim 16, **characterized in that** it additionally comprises the following steps:
G) inserting the front end (31) of a surgical tool (10) into one of the depressions (32) provided on the docking station (2), where the position of the bottom of the depression (32) with respect to the marking indicators (9) on the reference device (12a) of the docking station (2) is known; and
H) measuring the spatial position of the marking indicators (9) that are fastened to the docking stations (2) and to the surgical tool (10) through a position detecting device (14);
I) determining the spatial position of the front end (31) of the surgical tool (10) from the measured positions of all the marking indicators (9) through a computer (16).

## Revendications

1. Dispositif (1) d'étalonnage de dimensions géométriques d'instruments chirurgicaux (10) et de l'orientation de ceux-ci dans l'espace, comprenant
A) une station d'amarrage (2) présentant deux mâchoires (3) et une surface (21) ; et
B) au moins trois repères (9) disposés fixement sur la station d'amarrage (2) et dont la position dans l'espace est repérable de manière électromagnétique ou acoustique, qui servent à déterminer la position et l'orientation du dispositif (1) dans l'espace au moyen d'un dispositif de détection de position (14),
C) les mâchoires (3) sont fixes par rapport à la surface (21) de la station d'amarrage (2) et présentent chacune une paroi latérale (40), de sorte que la surface (21) et les parois latérales (40) des deux mâchoires (3) forment un passage en U (18) ; dans lequel
D) les deux mâchoires (3) sont conçues, au moins sur les parties de surface opposées, en forme de cylindre circulaire, d'hyperboloïde ou de double cône, de sorte qu'un instrument chirurgical (10) placé entre les mâchoires (3) peut être mis en appui transversalement à son axe longitudinal (11) sur au moins l'un des points d'appui de chaque mâchoire (3) ; et
E) les deux parois latérales (40) ou la surface (21) ont une forme qui comprend au moins deux autres points d'appui pour un instrument chirurgical (10) placé entre les mâchoires (3) transversalement à son axe longitudinal (11), de sorte qu'un amarrage latéral, défini par rapport aux repères (9), d'un instrument chirurgical (10) placé entre les mâchoires (3) est rendu possible ;
**caractérisé en ce que**
F) le dispositif (1) comprend un plateau tournant (6) présentant un axe de rotation (25) s'étendant transversalement à la surface (21) et deux mâchoires de serrage (5) dépassant de la surface (21).

2. Dispositif (1) selon la revendication 1, **caractérisé en ce que** les deux mâchoires (3) sont conçues, au moins sur leurs parties de surface opposées, en forme de double cône, renfermant au milieu de l'axe une encoche en V (13), et présentent un axe central (24) défini par les centres de cercle des aires de section du cône en forme de cercle ou de secteur de cercle.

3. Dispositif (1) selon la revendication 2, **caractérisé en ce que** les encoches (13) des deux mâchoires (3) ont une profondeur T et sont alignées l'une par rapport à l'autre.

4. Dispositif (1) selon la revendication 3, **caractérisé en ce que** l'angle α formé entre la droite de référence (4) traversant l'une des parois latérales (40) dans les encoches (13) à la profondeur T et coupant les axes centraux (24), et l'axe longitudinal (11) d'un instrument chirurgical (10) amarré est α < 90°.

5. Dispositif (1) selon l'une quelconque des revendications 1 à 4, **caractérisé en ce qu'**il comprend en plus des moyens de positionnement (30) pour positionner l'extrémité avant (31) de différents instruments chirurgicaux (10) par rapport à la station d'amarrage (2).

6. Dispositif (1) selon l'une quelconque des revendications 1 à 5, **caractérisé en ce que** les mâchoires (3) présentent l'une par rapport à l'autre une distance comprise entre au moins 100 mm et 300 mm.

7. Dispositif (1) selon l'une quelconque des revendications 1 à 6, **caractérisé en ce que** les mâchoires (3) présentent des axes centraux (24) qui sont parallèles l'un à l'autre.

8. Dispositif (1) selon l'une quelconque des revendications 1 à 7, **caractérisé en ce qu'**un ressort de torsion (26) est disposé au niveau de la station d'amarrage (2), ressort au moyen duquel le plateau tournant (6) peut être poussé dans un premier sens de rotation (27) autour de l'axe de rotation (25).

9. Dispositif (1) selon la revendication 8, **caractérisé en ce que** le plateau tournant (6) peut être déplacé contre la force du ressort dans un deuxième sens de rotation autour de l'axe de rotation (25) au moyen d'un élément de commande (8).

10. Dispositif (1) selon l'une quelconque des revendications 1 à 9, **caractérisé en ce que** les mâchoires de serrage (5) sont disposées sur le plateau tournant (6) de telle sorte qu'elles sont traversées au centre par une droite (7) coupant l'axe de rotation (25).

11. Dispositif (1) selon la revendication 10, **caractérisé en ce que**, lors d'une rotation du plateau tournant (6) dans le premier sens de rotation (27), l'angle β formé entre la droite (7) et la droite de référence (4) est réduit, de sorte qu'un instrument chirurgical (10) placé entre les mâchoires (3) peut être pressé contre les mâchoires (3).

12. Dispositif (1) selon l'une quelconque des revendications 9 à 11, **caractérisé en ce qu'**un engrenage (29) est disposé entre l'élément de commande (8) et le plateau tournant (6).

13. Dispositif (1) selon l'une quelconque des revendications 5 à 12, **caractérisé en ce que** les moyens de positionnement (30) comprennent des évidements (32) qui présentent une géométrie différente les uns des autres.

14. Dispositif (1) selon la revendication 13, **caractérisé en ce que** les évidements (32) présentent différentes aires de section transversale.

15. Dispositif (1) selon l'une quelconque des revendications 1 et 5 à 14, **caractérisé en ce que** la surface (21) est conçue sous une forme plane et les mâchoires (3) sont en forme de cylindre circulaire au moins dans les parties de surface opposées.

16. Procédé d'étalonnage de dimensions géométriques d'instruments chirurgicaux (10) et de l'orientation de ceux-ci dans l'espace au moyen du dispositif selon l'une quelconque des revendications 1 à 15, comprenant les étapes consistant à :
A) placer un instrument chirurgical (10) dans un passage en U (18), qui est formé par la surface (21) d'une station d'amarrage (2) et deux mâchoires (3) en forme de cylindre circulaire ou de double cône faisant partie de la station d'amarrage (2) ;
B) faire tourner l'instrument chirurgical (10) de telle sorte que l'angle α, qui est formé par l'axe longitudinal (11) de l'instrument chirurgical (10) et une droite de référence (4) définie par la forme des mâchoires (3) ou la forme des mâchoires (3) et la surface (21), est réduit jusqu'à ce que l'enveloppe de l'instrument chirurgical (10) vient s'appuyer sur les parois latérales (40) des mâchoires (3) dans une orientation définie par la forme des mâchoires (3) ou la forme des mâchoires (3) et la surface (21) ;
C) presser l'instrument chirurgical (10) contre les mâchoires (3) au moyen des mâchoires de serrage (5) disposées sur le plateau tournant (6) ;
D) mesurer la position spatiale des repères (9), qui sont fixés sur la station d'amarrage (2) et sur l'instrument chirurgical (10), au moyen d'un dispositif de détection de position (14) ;
E) déterminer l'angle α d'après les positions mesurées de tous les repères (9) au moyen d'un ordinateur (16) ; et
F) déterminer le diamètre s'étendant perpendiculairement aux axes centraux (24) des mâchoires (3) ou la largeur de l'instrument chirurgical (10) d'après l'angle mesuré α, la géométrie connue des mâchoires (3) par rapport aux repères (9) au niveau de la station d'amarrage (2), au moyen de l'ordinateur (16).

17. Procédé selon la revendication 16, **caractérisé en ce qu'**il comprend en outre les étapes suivantes consistant à :
G) insérer l'extrémité avant (31) d'un instrument chirurgical (10) dans l'un des évidements (32) situés sur la station d'amarrage (2), la position du fond de l'évidement (32) par rapport aux repères (9) sur le moyen de référence (12a) au niveau de la station d'amarrage (2) étant connue ; et
H) mesurer la position spatiale des repères (9), qui sont fixés sur la station d'amarrage (2) et sur l'instrument chirurgical (10), au moyen du dispositif de détection de position (14) ;
I) déterminer la position spatiale de l'extrémité avant (31) de l'instrument chirurgical (10) d'après les positions mesurées de tous les repères (9) au moyen de l'ordinateur (16).
